# EUROPEAN PATENT APPLICATION

(11) **EP 1 157 817 A2**
(43) Date of publication of application: **28.11.2001**
(21) Application number: 01850094.2
(22) Date of filing: 21.05.2001
(51) Int. Cl.: B31F 1/07, A61F 13/00

(54) **Method for producing a fluid-pervious fabric for imparting a pattern to a fibre web, such a fluid-pervious fabric, and such a fibre web**

(30) Priority: 24.05.2000 SE 0001948
(71) Applicant: SCA Hygiene Products AB, 405 03 Göteborg (SE)
(72) Inventor: Zawadzki, Valdemar, 438 34 Landvetter (SE); Falk, Magnus, 439 94 Onsala (SE); Mansson, Anna, 431 47 Mölndal (SE)
(74) Representative: Egeröd, Lisbeth

(57) **Abstract**

A method for producing a fluid-pervious fabric (301) for imparting a pattern to a fibre web. The fabric comprises at least one polymer material with a softening temperature, and a fabric structure (306). The fabric (301') is heated to a temperature higher than the softening temperature, wherein a forming pressure (Δp₃) is applied between a first surface (304) and a second surface (305) of the fabric. Fabric patterning members (309,309') on the support contribute to a deformation of the fabric structure (306) in the Z-direction in deformation zones (311) in which the fluid permeability remains essentially unchanged. The fabric (301) is cooled down to a temperature lower than the softening temperature rendering the deformation permanent. Furthermore, the invention relates to a fluid-pervious fabric, e.g. a wire or a felt, and patterned fibre web, e.g. a paper web, a nonwoven web, or another fibre web.

## Description

### Technical field

The present invention relates to a method for producing a fluid-pervious fabric for imparting a pattern to a fibre web.

Furthermore, the invention relates to a fluid-pervious fabric, e.g. a wire or a felt, produced by means of the method. The fluid-pervious fabric according to the invention is primarily intended for use as a patterning or shaping fabric in a through-air dryer, but can also be used for wet-forming, foam-forming, air-laying, hydroentangling and/or aperturing in order to impart a desired pattern to a fibre web.

The invention also relates to a fibre web, patterned by means of the fluid-pervious fabric, e.g. a paper web, a nonwoven web, or another fibre web for use in absorbent articles such as toilet paper, handwipes, industrial wipes, diapers, napkins, etc.

### Background of the invention

For different reasons, it can be desirable to create special patterns or indicia in a fibre web. Such patterns can be utilised in order to improve the aesthetic or tactile perception a user gets when observing, handling, or using an absorbent article comprising the fibre web. Suitable patterns can also be utilised in order to improve or enhance the physical properties, such as absorption properties, of an absorbent article.

Examples of articles where patterns of the above-mentioned type have been used previously are toilet paper, handwipes, industrial wipes, diapers, napkins, etc.

The patterning of fibre webs can be performed in a number of previously well-known ways, and can take place e.g. when forming, hydroentangling, pressing, shaping, drying or embossing a fibre web.

One previously well-known way of patterning a fibre web is to utilise a forming wire having metal threads or the like inserted into the wire structure in a desired pattern. During the forming, the wire will create so-called watermarks in the resulting fibre web.

Furthermore, it has been suggested previously to subject a papermaking wire or felt to an embossing process, wherein a desired pattern is imprinted by means of compressing the internal wire or felt structure in certain areas and leaving the structure unaffected in surrounding areas. Such an embossed fabric can be brought into contact with the fibre suspension or fibre web during the forming, pressing, drying, etc., in order to impart a pattern to the resulting fibre web.

Another previously known way of patterning a fibre web is to utilise a forming wire, a press felt or a drying fabric in which portions of the water-pervious wire structure have been partially or completely blocked by a chemical binder or the like.

Accordingly, the international patent application WO 98/53138 discloses a macroscopically mono-planar paper-making belt for a paper machine. The belt has a resinous framework with a web-side surface defining an X-Y-plane, a back-side surface opposite the web-side surface, a Z-direction perpendicular to the X-Y-plane, and discrete deflection conduits extending between the web-side and backside surfaces. Each conduit has an axis and walls, the axes of at least some of the conduits forming acute angles with the Z-direction.

According to WO 98/53138, the paper-making belt is made by means of disposing liquid photosensitive resin in a forming unit in order to form a coating having opposite surfaces and a pre-selected thickness. The forming unit is disposed in a first direction such that there is an acute angle between a first surface of the coating and the first direction, whereafter a mask having opaque regions and transparent regions defining a pre-selected pattern is positioned between the first surface and an apparatus for generating curing radiation. The unshielded portions but not the shielded portions of the coating are cured by exposing the coating to radiation via the mask in order to form a partially-formed belt. All uncured liquid photosensitive resin is subsequently removed from the belt leaving a hardened resinous structure defining the claimed belt. In WO 98/53138, also a paper web and a process of producing such a web by means of utilising the paper-making belt is disclosed.

Furthermore, it is previously known to impart a pattern to a fibre web in a through-air drying process by means of bringing the fibre web into contact with a special patterning or shaping fabric during the drying, or by means of drying the fibre web between two such fabrics.

Accordingly, the patent publication US 5,893,965 discloses a process for making a paper sheet, wherein a web is transferred from a forming wire to a papermaking belt preferably having deflection conduits. The web is overlaid with a flexible sheet of material such that the web is disposed intermediate the sheet of material and the papermaking belt. The flexible sheet has an air permeability less than the papermaking belt, and is preferably air-impermeable. An application of a fluid pressure differential to the sheet of material causes deflection of at least a portion of the sheet of material towards the papermaking belt and, preferably, deflection of at least a portion of the web into the conduits of the papermaking belt and water removal from the web through the conduits of the papermaking belt.

According to US 5,893,965, the disclosed process makes it possible to produce a paper sheet with e.g. a pattern of protruding domes, but with a more uniform basis weight and density distribution throughout the general plane of the web than what has previously been possible.

However, the previously known methods for producing different patterning fabrics, e.g. wires or felts, can be perceived as having some disadvantages.

Forming or shaping wires produced by means of adding materials such as photosensitive resin, can be sensitive to cleaning with wire showers and wire detergents. Accordingly, there is a great risk that such an added material is washed off, so that the patterning ability gradually disappears.

Previously known methods for producing patterning fabrics by means of methods which reduce the fluid permeability in the treated areas of the fabric, will provide forming fabrics which can cause runnability problems, since the obtainable fabric patterns result in fibre webs with local areas of a much lower grammage than the surrounding areas and, consequently, also with a lower strength. These "weak spots" can cause web breaks and impair the runnability of a paper or nonwoven machine, or otherwise cause an inferior product quality due to holes or linting problems.

The previously utilised shaping fabrics in through-air dryers often exhibit a lower air permeability in the fabric areas intended to produce the pattern. This will create undesired "wet spots" in the dried fibre web.

### Summary of the invention

Accordingly, a first object of the present invention is to provide a method of producing a fluid-pervious fabric for imparting a pattern to a fibre web, which fibre web can be produced with an excellent quality and runnability, since the resulting pattern in the fibre web does not have any weak and wet spots, and since the fluid-pervious fabric exhibits an excellent durability which reduces downtime in the fibre web production.

In accordance with claim 1, this first object is achieved by means of the method utilising a fluid-pervious fabric comprising at least one polymer material with a softening temperature, a first surface, a second surface opposite the first surface, and a fabric structure comprising a plurality of channels providing fluid permeability between the first and the second surfaces. The second surface is brought into contact with an essentially; rigid support having a fabric-contacting side with first fabric patterning members in a desired configuration and a backside opposite the fabric-contacting side. Thereby, the fluid-pervious fabric is heated to a temperature higher than the softening temperature, and a forming pressure is applied between the first surface and the second surface so that the first fabric patterning members contribute to a deformation of the fabric structure in the Z-direction in deformation zones in which the fluid permeability remains essentially unchanged, whereafter the fabric is cooled down to a temperature lower than the softening temperature in order to render the deformation in the Z-direction permanent.

A second object of the present invention is to provide a fluid-pervious fabric for imparting a pattern to a fibre web, which fabric has been obtained by means of the method according to the invention.

In accordance with claim 15, the second object is achieved by means of the fluid-pervious fabric comprising at least one polymer material with a softening temperature, a first surface, a second surface opposite the first surface, and a fabric structure comprising a plurality of channels providing fluid permeability between the first and the second surface. Thereby, the fluid-pervious fabric exhibits a permanent deformation of the fabric structure in the Z-direction in deformation zones in which the fluid permeability is essentially equal to the fluid permeability in fabric zones outside the deformation zones.

A third object of the present invention is to provide a patterned fibre web which has been given a desired pattern by means of the fluid-pervious fabric according to the invention.

In accordance with claim 18, this third object is achieved by means of the patterned fibre web comprising a plurality of fibres arranged in a fibre structure having a grammage and a porosity. Thereby, the fibre web exhibits a deformation of the fibre structure in the Z-direction in deformation zones, wherein the grammage and the porosity within the deformation zones are essentially equal to the grammage and porosity outside the deformation zones.

Further objects of the present invention will become apparent from the following description, and the technical features making it possible to achieve these further objects are defined in the dependent claims.

### Brief description of the drawings

In the following, the present invention will be described in greater detail with reference to the attached drawings, in which
- Fig. 1: schematically illustrates a first embodiment of the method according to the invention, seen across the X-direction,
- Fig. 2: schematically illustrates a second embodiment of the method according to the invention, seen across the X-direction,
- Fig. 3: schematically illustrates a third, preferred embodiment of the method according to the invention, seen across the X-direction,
- Fig. 4: is a schematic perspective view seen from a first side of a fluid-pervious fabric according to a preferred embodiment of the invention, wherein the X, Y and Z-directions of the fabric are indicated with (X), (Y) and (Z), and
- Fig. 5: is a schematic perspective view seen from a first side of a patterned fibre web according to the invention, wherein the fibre web has been patterned on the fluid-pervious fabric in Fig. 4, and the X, Y and Z-directions of the fibre web are indicated with (X), (Y) and (Z).

### Detailed description of preferred embodiments

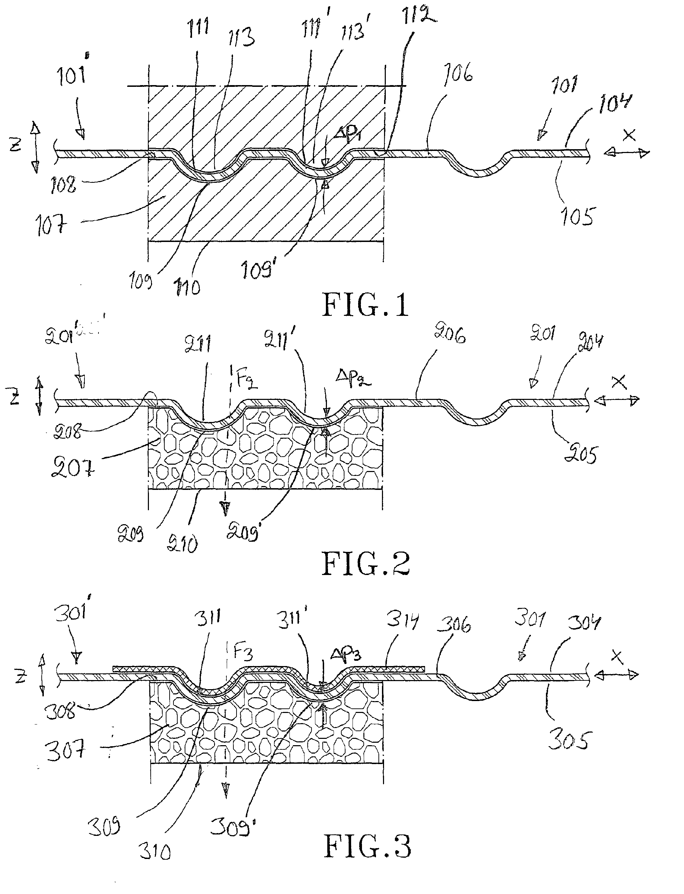

In the following, a number of advantageous and preferred embodiments of the method according to the invention will be described in greater detail, and when applicable with reference to Figs. 1- 5.

The method according to the invention is intended for producing a fluid-pervious fabric 101; 201; 301; 401 for imparting a pattern 502, 502', 502" to a fibre web 503. The fluid-pervious fabric comprises at least one polymer material with a softening temperature, a first surface 104: 204; 304; 404, a second surface 105: 205; 305; 405 opposite the first surface, and a fabric structure 106: 206; 306; 406 comprising a plurality of channels providing fluid permeability between the first and the second surfaces. Thereby, the fluid-pervious fabric can be of any suitable type, e.g. a papermaking wire of a plastic material, a papermaking felt comprising synthetic polymer fibres, or another suitable fluid-pervious membrane.

The second surface 105: 205; 305; 405 is brought into contact with an essentially rigid support 107: 207; 307, having a fabric-contacting side 108: 208; 308 with first fabric patterning members 109, 109': 209, 209'; 309, 309' in a desired configuration and a backside 110: 210; 310 opposite the fabric-contacting side.

According to the invention, the fluid-pervious fabric 101'; 201'; 301' is heated to a temperature higher than the softening temperature, wherein a forming pressure Δp₁; Δp₂; Δp₃ is applied between the first surface 104: 204; 304 and the second surface 105; 205; 305, so that the first fabric patterning members 109, 109': 209, 209'; 309, 309' contribute to a deformation of the fabric structure 106: 206; 306; 406 in the Z-direction in deformation zones 111, 111', 111"; 211; 311; 411, 411', 411" in which the fluid permeability remains essentially unchanged. Thereafter, the fluid-pervious fabric 101; 201; 301; 401 is cooled down to a temperature lower than the softening temperature in order to render the deformation in the Z-direction permanent.

In a first embodiment of the method according to the invention, illustrated in Fig. 1, the above-mentioned forming pressure Δp₁ is created by means of first and second press surfaces 108, 112 arranged in order to form a press nip. In this embodiment, the first fabric patterning members 109, 109' are provided on the first press surface 108 being part of the support 107, and are cooperating with inverse, second patterning members 113, 113' on the second press surface 112. Thereby, advantageously but not necessarily, at least one of the press surfaces 108, 112 is provided on a rotatable embossing roll. Furthermore, the second fabric patterning members 113, 113' are advantageously provided on a single point deforming means programmed for generating the deformation in a chosen configuration across at least one of the surfaces of the fluid-pervious fabric contacting the support. Heating means (not shown in the drawings) are advantageously provided in at least one of the press surfaces 108, 112 and heat said fabric structure 106 at least in positions intended to become the deformation zones 111, 111'. The fluid-pervious fabric can advantageously be preheated before the deformation in the Z-direction.

In an alternative embodiment of the method according to the invention (not shown in the drawings), the forming pressure is created at least partially by means of controlling the pressure in a fluid provided on at least one side of a flexible and elastic, fluid-impermeable membrane. In this embodiment, the fluid-impermeable membrane in cooperation with the first fabric patterning members of the support causes the deformation in the Z-direction of the fluid-pervious fabric.

In a second advantageous embodiment ofthe method according to the invention, illustrated in Figs. 2 and 3, also the support 207; 307 is fluid-pervious, wherein a fluid of a higher temperature than the above-mentioned softening temperature is passed through the channels of the fluid-pervious fabric 201'; 301' and through the support 207; 307 at a flow rate (F₂; F₃) sufficient for generating the forming pressure Δp₂; Δp₃ and causing the deformation in the Z-direction. This embodiment provides the additional advantage that the fluid passed through the fluid-pervious fabric ensures that the channels in the fabric remain open also after the forming.

In a third, preferred embodiment of the method according to the invention, illustrated in Fig. 3, a flexible and elastic membrane 314 with a lower fluid permeability than the above-mentioned fabric structure is brought into contact with the first surface 304 of the fluid-pervious fabric 301'. In this embodiment, the fluid is passed through the membrane 314, the fabric 301' and the support 307, wherein the membrane 314 provides a contribution to the forming pressure Δp₃.

In the second and third embodiments of the method according to the invention, the support advantageously comprises a heating zone in which the fluid is added for heating the fabric and creating the forming pressure.

Furthermore, the support particularly advantageously comprises a cooling zone subsequent to the heating zone. The fluid-pervious fabric is even more advantageously heated with heated air before the deformation in the Z-direction, whereafter the fluid-pervious fabric is cooled down with cooled air in order to render the deformation permanent. The support is advantageously a rotatable, cylindrical roll, and/or comprises a sintered metallic material or a metal wire.

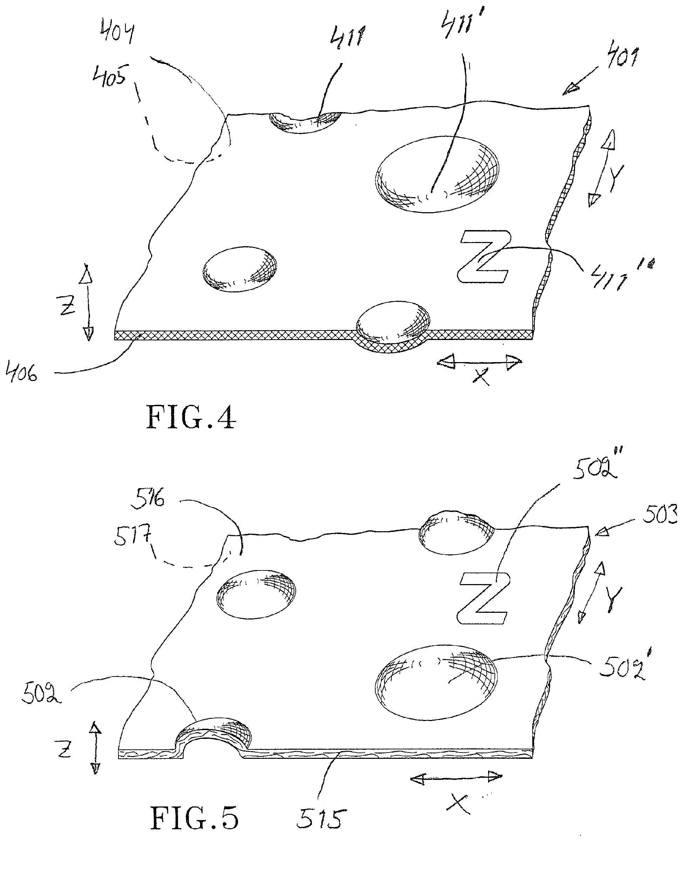

In the following, a preferred embodiment of a fluid-pervious fabric for imparting a pattern to a fibre web according to the invention will be described with reference to Fig. 4.

The fluid-pervious fabric 401 comprises at least one polymer material with a softening temperature, a first surface 404, a second surface 405 opposite the first surface, and a fabric structure 406 comprising a plurality of channels providing fluid permeability between the first and the second surface.

According to the invention and the preferred embodiment, the fluid-pervious fabric 401 exhibits a permanent deformation of the fabric structure 406 in the Z-direction in deformation zones 411, 411', 411" in which the fluid permeability is essentially equal to the fluid permeability in fabric zones outside the deformation zones.

In the preferred embodiment, the polymer material exhibits portions which have been softened and subsequently solidified in the deformation zones 411, 411', 411".

In a particularly preferred embodiment, the fluid-pervious fabric 401 exhibits the deformation in a chosen configuration across at least one of the surfaces 404, 405.

In the following, a preferred embodiment of a patterned fibre web according to the invention will be described with reference to Fig. 5.

The fibre web 503 illustrated in Fig. 5 has been through-air dried in contact with the fluid-pervious fabric shown in Fig. 4 in a conventional through-air dryer section.

The fibre web 503 comprises a plurality of fibres arranged in a fibre structure 515 having a grammage and a porosity. Thereby, the fibre web can be of any suitable type, e.g. a paper web, a board web, a nonwoven web, or another fibre web. Furthermore, the fibres constituting the fibre web can be of any suitable type, e.g. cellulose pulp fibres and/or natural or manmade fibres based on natural or synthetic polymers.

According to the invention and the preferred embodiment, the fibre web 503 exhibits a deformation of the fibre structure 515 in the Z-direction in deformation zones 502, 502', 502", wherein the grammage and the porosity within the deformation zones are essentially equal to the grammage and the porosity outside the deformation zones.. Accordingly, except for the above-mentioned deformation in the Z-direction, the fibre structure of the fibre web according to the invention has remained virtually unaffected after the patterning imparted by the fluid pervious fabric according to the invention.

As has become evident from the foregoing, it is also conceivable with advantageous embodiments of the invention in which the fibre web has been imparted the pattern in another way than by means of through-air drying, e.g. by means of forming, pressing, hydroentangling or embossing the fibre web in contact with at least one fluid-pervious fabric according to the invention.

In a particularly advantageous embodiment of the patterned fibre web according to the invention, the deformation zones 502, 502', 502" are visible as a chosen pattern across both surfaces 516, 517 of the fibre web 503.

The patterned fibre web 503 according to the invention has advantageously been wet-formed or foam-formed. However, it is also conceivable with embodiments in which the fibre web has been air-laid.

Furthermore, its also conceivable with advantageous embodiments in which the fibre web has been hydraulically apertured or entangled.

In a particularly advantageous embodiment of the patterned fibre web according to the invention, it has been through-air dried (TAD).

Accordingly, the pattern 502, 502', 502" in the fibre web 503 according to the invention has been created by means of forming and/or patterning/aperturing on, and/or shaping and/or drying in contact with at least one fluid-pervious fabric 401 according to the invention.

The present invention should by no means be regarded as being limited to what has been described above in connection with the different embodiments, or to what is shown in the attached drawings, but the scope of the invention is defined in the appended claims.

## Claims

1. A method for producing a fluid-pervious fabric (101; 201; 301; 401) for imparting a pattern (502, 502', 502") to a fibre web (503), said fabric comprising at least one polymer material with a softening temperature, a first surface (104: 204; 304; 404), a second surface (105: 205; 305; 405) opposite said first surface, and afabric structure (106: 206; 306; 406) comprising a plurality of channels providing fluid permeability between said first and said second surfaces, wherein said second surface (105: 205; 305; 405) is brought into contact with an essentially rigid support (107:207;307) having a fabric-contacting side (108:208; 308) with first fabric patterning members (109, 109': 209, 209'; 309, 309') in a desired configuration and a backside (110: 210; 310) opposite said fabric-contacting side,
**characterized in that** the fluid-pervious fabric (101'; 201'; 301') is heated to a temperature higher than said softening temperature, and that a forming pressure (Δp₁; Δp₂; Δp₃) is applied between said first surface (104: 204; 304) and said second surface (105: 205; 305), so that said first fabric patterning members (109, 109': 209, 209'; 309, 309') contribute to a deformation of said fabric structure (106: 206; 306; 406) in the Z-direction in deformation zones (111, 111', 111"; 211; 311; 411, 411', 411") in which said fluid permeability remains essentially unchanged, whereafter said fluid-pervious fabric (101; 201; 301; 401) is cooled down to a temperature lower than said softening temperature in order to render said deformation in the Z-direction permanent.

2. A method according to claim 1,
**characterized in that** the forming pressure (Δp₁) is created by means of first and second press surfaces (108, 112) arranged in order to form a press nip, wherein said first fabric patterning members (109, 109') are provided on said first press surface (108) being part of said support (107), and are cooperating with inverse, second patterning members (113, 113') on said second press surface (112).

3. A method according to claim 1 or 2,
**characterized in that** heating means are provided in at least one of said press surfaces (108, 112) and heat said fabric structure (106) at least in positions intended to become said deformation zones (111, 111').

4. A method according to claim 2 or 3,
**characterized in that** the fluid-pervious fabric is preheated before said deformation in the Z-direction.

5. A method according to any one of claims 2 - 4,
**characterized in that** at least one of said press surfaces (108, 112) is provided on a rotatable embossing roll.

6. A method according to any one of claims 2 - 5,
**characterized in that** the second fabric patterning members (113, 113') are provided on a single point deforming means programmed for generating said deformation in a chosen configuration across at least one of said surfaces of said fluid-pervious fabric contacting said support.

7. A method according to claim 1,
**characterized in that** the forming pressure is created at least partially by means controlling the pressure in a fluid provided on at least one side of a flexible and elastic, fluid-impermeable membrane, which in cooperation with said first fabric patterning members causes said deformation in the Z-direction of the fluid-pervious fabric.

8. A method according to claim 1,
**characterized in that** also said support (207; 307) is fluid-pervious, wherein a fluid of a higher temperature than said softening temperature is passed through said channels of said fluid-pervious fabric (201'; 301') and through said support (207; 307) at a flow rate (F₂; F₃) sufficient for generating said forming pressure (Δp₂; Δp₃) and causing said deformation in the Z-direction.

9. A method according to claim 8,
**characterized in that** a flexible and elastic membrane (314) with a lower fluid permeability than said fabric structure is brought into contact with said first surface (304) of said fluid-pervious fabric (301'), and that said fluid is passed through said membrane (314), said fabric (301') and said support (307), wherein said membrane (314) provides a contribution to said forming pressure (Δp₃).

10. A method according to any one of claims 7 - 9,
**characterized in that** the support comprises a heating zone in which said fluid is added for heating said fabric and creating said forming pressure.

11. A method according to any one of claims 7 - 10,
**characterized in that** the support comprises a cooling zone subsequent to said heating zone.

12. A method according to any one of claims 7 - 11,
**characterized in that** the fluid-pervious fabric is heated with heated air before said deformation in the Z-direction, and that the fluid-pervious fabric thereafter is cooled down with cooled air in order to render said deformation permanent.

13. A method according to any one of claims 7 - 12,
**characterized in that** the support is a rotatable, cylindrical roll.

14. A method according to any one of claims 7 - 13,
**characterized in that** the support comprises a sintered metallic material or a metal wire.

15. A fluid-pervious fabric for imparting a pattern to a fibre web, said fabric (401) comprising at least one polymer material with a softening temperature, a first surface (404), a second surface (405) opposite said first surface, and a fabric structure (406) comprising a plurality of channels providing fluid permeability between said first and said second surface, **characterized in that** the fluid-pervious fabric (401) exhibits a permanent deformation of said fabric structure (406) in the Z-direction in deformation zones (411, 411', 411") in which said fluid permeability is essentially equal to the fluid permeability in fabric zones outside said deformation zones.

16. A fluid-pervious fabric according to claim 15,
**characterized in that** the polymer material exhibits portions which have been softened and subsequently solidified in the deformation zones (411, 411', 411").

17. A fluid-pervious fabric according to claim 15 or 16,
**characterized in that** the fluid-pervious fabric (401) exhibits said deformation in a chosen configuration across at least one of said surfaces (404, 405).

18. A patterned fibre web, comprising a plurality of fibres arranged in a fibre structure (515) having a grammage and a porosity, **characterized in that** the fibre web (503) exhibits a deformation of said fibre structure (515) in the Z-direction in deformation zones (502, 502', 502"), wherein the grammage and the porosity within said deformation zones are essentially equal to the grammage and the porosity outside the deformation zones.

19. A patterned fibre web according to claim 18,
**characterized in that** the deformation zones (502, 502', 502") are visible as a chosen pattern across both surfaces (516, 517) of said fibre web (503).

20. A patterned fibre web according to claim 18 or 19,
**characterized in that** the fibre web (503) has been wet-formed or foam-formed.

21. A patterned fibre web according to claim 18 or 19,
**characterized in that** the fibre web has been air-laid.

22. A patterned fibre web according to any one of claims 18 - 21,
**characterized in that** the fibre web has been hydraulically apertured or entangled.

23. A patterned fibre web according to any one of claims 18 - 22,
**characterized in that** the fibre web has been through-air dried (TAD).

24. A patterned fibre web according to any one of claims 18 - 23,
**characterized in that** the pattern (502, 502', 502") in the fibre web (503) has been created by means of forming and/or patterning/aperturing on, and/or drying or shaping in contact with at least one fluid-pervious fabric (401) according to any one of claims 15 - 17.
